# EUROPÄISCHE PATENTSCHRIFT

(11) **EP 0 980 251 B1**
(45) Veröffentlichungstag und Bekanntmachung des Hinweises auf die Patenterteilung: **21.08.2002**
(21) Anmeldenummer: 98933500.5
(22) Anmeldetag: 08.05.1998
(51) Int. Cl.: A61K 38/00, A61P 17/02

(54) **TISSUE-FAKTOR ZUR BEEINFLUSSUNG VON GEFÄSSBILDUNG**
TISSUE FACTOR FOR INFLUENCING BLOOD VESSEL FORMATION
THROMBOPLASTINE TISSULAIRE DESTINEE A INFLUENCER LA FORMATION VASCULAIRE

(30) Priorität: 09.05.1997 DE 19719652; 07.05.1998 WO PCT/DE98/01278
(43) Veröffentlichungstag der Anmeldung: 23.02.2000
(73) Patentinhaber: MERCKLE GMBH, D-89079 Ulm (DE)
(72) Erfinder: NAWROTH, Peter, D-69207 Sandhausen (DE); NAKAGAWA, Katsumi, Kita-ku Kyoto 603 (JP); ZHANG, Youming, D-69117 Heidelberg (DE)
(74) Vertreter: Schüssler, Andrea, Dr.
(86) Internationale Anmeldenummer: DE9801306
(87) Internationale Veröffentlichungsnummer: WO98051321

(56) Entgegenhaltungen:
- WO-A-94/05328
- WO-A-98/34634
- Y. ZHANG ET AL.: "TISSUE FACTOR CONTROLS THE BALANCE OF ANGIOGENIC AND ANTIANGIOGENIC PROPERTIES OF TUMOR CELLS IN MICE." THE JOURNAL OF CLINICAL INVESTIGATION, Bd. 94, Nr. 3, September 1994, Seiten 1320-1327, XP002081086 NEW YORK, N.Y., US
- M. SHOJI ET AL.: "TISSUE FACTOR (TF) REGULATES THE EXPRESSION OF VASCULAR ENDOTHELIAL GROWTH FACTOR (VEGF) IN VITRO AND AGIOGENESIS IN VIVO." BLOOD, Bd. 88, Nr. 10 SUPPL 1 (PART 1 OF 2), 15. November 1996, Seite 514a XP002081087 NEW YORK, N.Y., US
- M. SHOJI ET AL.: "MOLECULAR MECHANISMS LINKING THROMBOSIS AND ANGIOGENESIS IN CANCER." TRENDS IN CARDIOVASVULAR MEDICINE, Bd. 7, Nr. 2, Februar 1997, Seiten 52-59, XP002081088 NEW YOK, N.Y., US
- P. CARMELIET ET AL.: "ROLE OF TISSUE FACTOR IN EMBRYONIC BLOOD VESSEL DEVELOPMENT." NATURE, Bd. 383, 5. September 1996, Seiten 73-75, XP002081089 LONDON GB
- J. CONTRINO ET AL.: "IN SITU DETECTION OF TISSUE FACTOR IN VASCULAR ENDOTHELIAL CELLS: CORRELATION WITH THE MALIGNANT PHENOTYPE OF HUMAN BREAST DISEASE." NATURE MEDICINE., Bd. 2, Nr. 2, Februar 1996, Seiten 209-215, XP002081090 NEW ORK, N.Y., US
- J.N. WILCOX ET AL.: "LOCALIZATION OF TISSUE FACTOR IN THE NORMAL VESSEL WALL AND IN THE ATHEROSCLEROTIC PLAQUE." PROCEEDINGS OF THE NATIONAL ACADEMY OF SCIENCES OF USA, Bd. 86, April 1989, Seiten 2839-2843, XP002081091 WASHINGTON US
- K. NAKAGAWA ET AL.: "THE ANGIOGENIC EFFECT OF TISSUE FCTOR ON TUMORS ND WOUNDS." SEMINARS IN THRMBOSIS AND HEMOSTASIS, Bd. 24, Nr. 3, 1998, Seiten 207-210, XP002081092 NEW YORK, N.Y., US

## Beschreibung

Die vorliegende Erfindung betrifft die Verwendung von Tissue-Faktor zur Beeinflussung von Gefäßbildung, insbesondere zur Aktivierung von Gefäßbildung, ganz besonders bei Wundheilung.

Mittels Gefäßen wird der Körper mit Blut versorgt. Gefäße umfassen Endothelund glatte Muskelzellen. Bei vielen Erkrankungen sind Gefäße bzw. deren Bildung beeinträchtigt. Solches findet sich z.B. bei gestörter Wundheilung, wie bei Diabetes mellitus, Vaskulitis, arterieller Verschlußkrankheit, chronischem venösen und infiziertem Ulcus. Auch gibt es große Probleme mit Wundheilung bei Innervationsstörungen, wie Paraplegie, Lepra, Neuropathie, etc., und Dekubitus von Pflegebedürftigen. Ferner sind Nahtschwächen und Wundheilungsstörungen bei Operationen, insbesondere des Darmes bzw. Transplantationen von Haut oder anderen Organen, bekannt. Bisher gibt es keine zufriedenstellenden Mittel, um bei Gefäßerkrankungen, insbesondere gestörter Wundheilung, eingreifen zu können.

Der vorliegenden Erfindung liegt somit die Aufgabe zugrunde, ein Mittel bereitzustellen, mit dem vorstehendes erreicht werden kann.

Erfindungsgemäß wird dies durch die Gegenstände in den Patentansprüchen erreicht.

Gegenstand der vorliegenden Erfindung ist somit die Verwendung von Tissue-Faktor zur Herstellung eines Arzneimittels zur Beeinflussung von Gefäßbildung, insbesondere zur Aktivierung von Gefäßbildung, ganz besonders bei Wundheilung.

Die vorliegende Erfindung beruht auf der Erkenntnis des Anmelders, daß Tissue-Faktor in Wunden von Tieren zur Bildung von Gefäßen (Blutgefäßen) führt. Er hat gefunden, daß die Gefäße Endothel- und glatte Muskelzellen aufweisen. Ferner hat er erkannt, daß mittels Tissue-Faktor Wundheilung erreicht werden kann. Des weiteren hat er gefunden, daß durch Inhibierung von Tissue-Faktor Gefäßbildung verhindert werden kann.

Tissue-Faktor ist ein Transmembran-Glykoprotein, das die Blutgerinnungsfaktoren VII bzw. VIIa bindet. Durch diese Bindung wird eine Aktivierung der Blutgerinnungsfaktoren X bzw. IX bewirkt, wodurch die Blutgerinnung über den extrinsischen bzw. intrinsischen Weg in Gang gesetzt wird. Tissue-Faktor hat ein Molekulargewicht von 43-46 kD. Seine Primärstruktur ist bekannt, ebenso das Gen für Tissue-Faktor und dessen Lokalisierung auf dem Chromosom (vgl. Scarpati, E.M., et al., Biochemistry 26, (1987), 5234-5238).

Erfindungsgemäß wird Tissue-Faktor zur Aktivierung von Gefäßbildung, insbesondere bei Wundheilung, verwendet. Der Ausdruck "Tissue-Faktor" betrifft einen Tissue-Faktor jeglicher Art und Herkunft. Es kann ein tierischer oder menschlicher Tissue-Faktor sein. Er kann glykosyliert oder nicht-glykosyliert sein. Auch kann es ein Fragment von Tissue-Faktor sein, das die Fähigkeit hat, Gefäße, insbesondere bei Wundheilung, zu bilden. Der Tissue-Faktor kann eine Wildtyp-Sequenz aufweisen. Auch kann seine Sequenz gegenüber der Wildtyp-Sequenz durch eine oder mehrere Aminosäuren verschieden sein. Ferner kann der Tissue-Faktor Teil eines Fusionsproteins sein.

In bevorzugter Ausführungsform liegt der Tissue-Faktor in Form einer exprimierbaren Nukleinsäure vor. Diese kann eine DNA und/oder RNA sein, wobei eine DNA, insbesondere eine genomische oder cDNA bzw. Fragmente davon, bevorzugt sind. Die vorstehenden Ausführungen hinsichtlich des Tissue-Faktor gelten hier entsprechend für die Nukleinsäure.

Die Expression der Nukleinsäure kann in üblicher Weise erreicht werden. Günstig kann es sein, wenn die Nukleinsäure, z.B. als DNA, insbesondere cDNA, in einem Vektor vorliegt, der zur Expression in tierischen Zellen geeignet ist. Solche Expressionsvektoren sind dem Fachmann bekannt. Beispielsweise können es Virus- oder Plasmid-Vektoren sein. Vorteilhaft ist es, wenn die Vektoren nicht in das Genom von Zellen integrieren, sondern episomal in den Zellen verbleiben. Damit wird eine transiente Expression des Tissue-Faktor erreicht, was bevorzugt ist. Ferner kann die Nukleinsäure als DNA, insbesondere cDNA, unter der Kontrolle eines konstitutiven oder induzierbaren Promotors stehen. Ein induzierbarer Promotor kann z.B. Gewebe-, Organ- und/oder Tumor-spezifisch sein. Günstig kann es sein, wenn die Nukleinsäure als DNA, insbesondere cDNA, unter der Kontrolle des CMV-Promotors z.B. in dem Expressionsvektor pcDNA3 (Invitrogen), oder unter der Kontrolle des SV40 Promotors, z.B. in dem Expressionsvektor pSVK3 (Pharmacia) vorliegt. Solche mit pcDNA3-TF (Tissue-Faktor) bzw. pSVK3-TF bezeichnete Expressionsplasmide sind ebenfalls Gegenstand der vorliegenden Erfindung. Besonders vorteilhaft kann es sein, wenn die Nukleinsäure als DNA, insbesondere cDNA, in einem Sindbis-Virus-Replikon-Vektor vorliegt. Ein solcher Vektor erlaubt eine extrem hohe Expression der Nukleinsäure. Ein Beispiel eines solchen Vektors ist das ELVS-Vektor-System von Viagene Inc.. Ein mit ELVS-TF (Tissue-Faktor) bezeichnetes Expressionsplasmid ist ebenfalls Gegenstand der vorliegenden Erfindung. Zur Herstellung eines vorstehenden Vektors wird der Fachmann auf bekannte Verfahren zurückgreifen. Ergänzend wird auf Maniatis, T., et al., Molecular Cloning, A Laboratory Manual, Cold Spring Harbor Laboratory, 1982 verwiesen.

Erfindungsgemäß wird Tissue-Faktor zur Aktivierung von Gefäßbildung, insbesondere bei Wundheilung, verwendet. Der Begriff "Gefäßbildung" betrifft eine Gefäßbildung jeglicher Art und an jeglicher Stelle. Beispielsweise betrifft es eine Gefäßbildung, um beeinträchtigte, z.B. alte, Gefäße zu ersetzen. Solche können z.B. im Gehirn oder Herz vorliegen, wodurch ein Schlaganfall bzw. Infarkt verhindert oder behandelt werden kann. Auch kann einer Altersdemenz vorgebeut werden. Ferner betrifft es eine Gefäßbildung bei der Behandlung von Arteriosklerose, M. Crohn und C. ulcerosa, diabetischer Retinopathie und tiefer Beinvenenthrombose/Ulcus cruris sowie der Verhinderung von Rezidiven. Insbesondere betrifft es eine Gefäßbildung und Wundheilung. Der Ausdruck "Wundheilung" betrifft eine Wundheilung jeglicher Art und an jeglicher Stelle. Es kann normale und gestörte Wundheilung sein. Letztere findet sich insbesondere bei Erkrankungen, wie Diabetes mellitus, Vaskulitis, arterieller Verschlußkrankheit, chronischem venösen und/oder infiziertem Ulcus sowie schlecht heilendem Magenulcus. Auch findet sich eine gestörte Wundheilung bei Innervationsstörungen, wie Paraplegie, Lepra, Neuropathie, etc., und Dekubitus bei Pflegebedürftigen. Des weiteren liegt eine gestörte Wundheilung vor, wenn Nahtschwächen und Heilungsstörungen nach Operationen, insbesondere des Darms und Transplantationen der Haut bzw. anderer Organe, auftreten. Darüberhinaus findet sich eine gestörte Wundheilung bei Knochenfrakturen, Verbrennungen und Behandlungen mit Steroiden.

Erfindungsgemäß wird Tissue-Faktor in Form eines Proteins oder einer exprimierbaren Nukleinsäure zur Aktivierung von Gefäßbildung, insbesondere bei Wundheilung, verabreicht. Günstig kann es sein, den Tissue-Faktor in Kombination mit weiteren die Gefäßbildung, insbesondere bei Wundheilung, fördernden Faktoren, wie z.B. "vascular endothelial growth factor" (VEGF), zu verabreichen. Diese Faktoren können ebenfalls in Form von Proteinen und/oder exprimierbaren Nukleinsäuren vorliegen. Die Verabreichung von Tissue-Faktor und den genannten Faktoren kann gleichzeitig oder nacheinander erfolgen. Die Art der Verabreichung von Tissue-Faktor alleine bzw. zusammen mit den genannten Faktoren kann sich am Wirkungsort, d.h. an der Stelle, wo eine Gefäßbildung, insbesondere bei Wundheilung, erfolgen soll, orientieren. Beispielsweise bietet es sich an, eine Stelle auf der Körperoberfläche lokal und eine im Inneren des Körpers systemisch zu behandeln. Zur Verabreichung von Tissue-Faktor alleine bzw. zusammen mit den genannten Faktoren können übliche Verfahren verwendet werden. Für die lokale Verabreichung ist es z.B. günstig, den oder die Faktoren in Liposomen zu verpacken oder auf Träger, insbesondere Goldpartikel, zu absorbieren und die Liposomen an der entsprechenden Stelle des Körpers aufzutragen bzw. die Träger, insbesondere Goldpartikel, in das Gewebe hineinzuschießen. Ferner werden für die Verabreichung von Tissue-Faktor alleine bzw. zusammen mit den genannten Faktoren pharmazeutische Zusammensetzungen bereitgestellt, die übliche Hilfsstoffe, wie Träger, Lösungsmittel, etc. enthalten können. Solche Zusammensetzungen sind ebenfalls Gegenstand der vorliegenden Erfindung.

Erfindungsgemäß wird Tissue-Faktor auch zur Inhibierung von Gefäßbildung verwendet. Hierzu kann der Tissue-Faktor in Form eines ihn inhibierenden Antikörpers vorliegen, ebenso kann der Tissue-Faktor in Form einer Nukleinsäure vorliegen, die eine "antisense"-Wirkung auf die Expression von Tissue-Faktor hat. Durch die Inhibierung von Gefäßbildung können insbesondere Tumorerkrankungen behandelt werden.

Mit der vorliegenden Erfindung ist es möglich, Gefäßbildung zu beeinflussen. Insbesondere kann Gefäßbildung aktiviert werden. Die erhaltenen Gefäße umfassen Endothel- und glatte Muskelzellen. Somit eignet sich die vorliegende Erfindung zur Vorbeugung und Behandlung der verschiedensten Erkrankungen. Beispiele solcher sind vorstehend angegeben. Insbesondere eignet sich die vorliegende Erfindung zur Behandlung und/oder Prophylaxe von Wundheilungsstörungen, ganz besonders bei Diabetes mellitus, wo es möglich ist, große, offene Wunden an den Extremitäten zu heilen. Desweiteren kann mit der vorliegenden Erfindung eine Gefäßbildung inhibiert werden. Somit eignet sich die vorliegende Erfindung auch, Erkrankungen, wie Tumorerkrankungen, zu behandeln. Die vorliegende Erfindung liefert einen großen Beitrag zur modernen Medizin.

### Kurze Beschreibung der Zeichnung

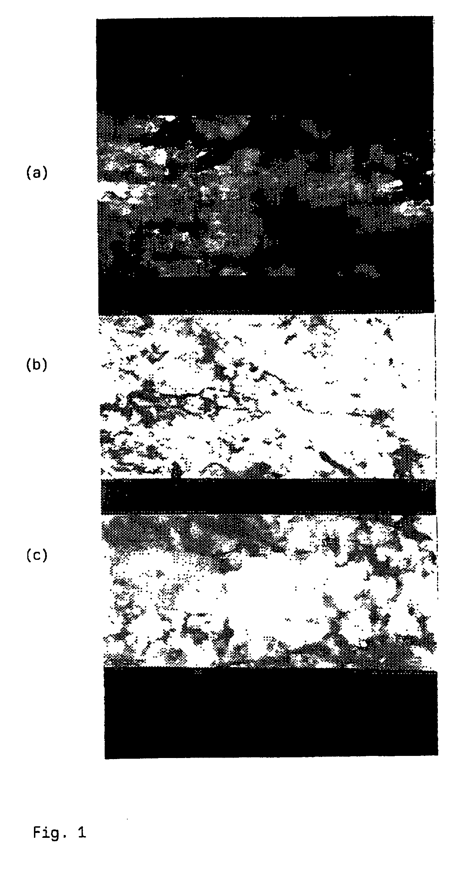

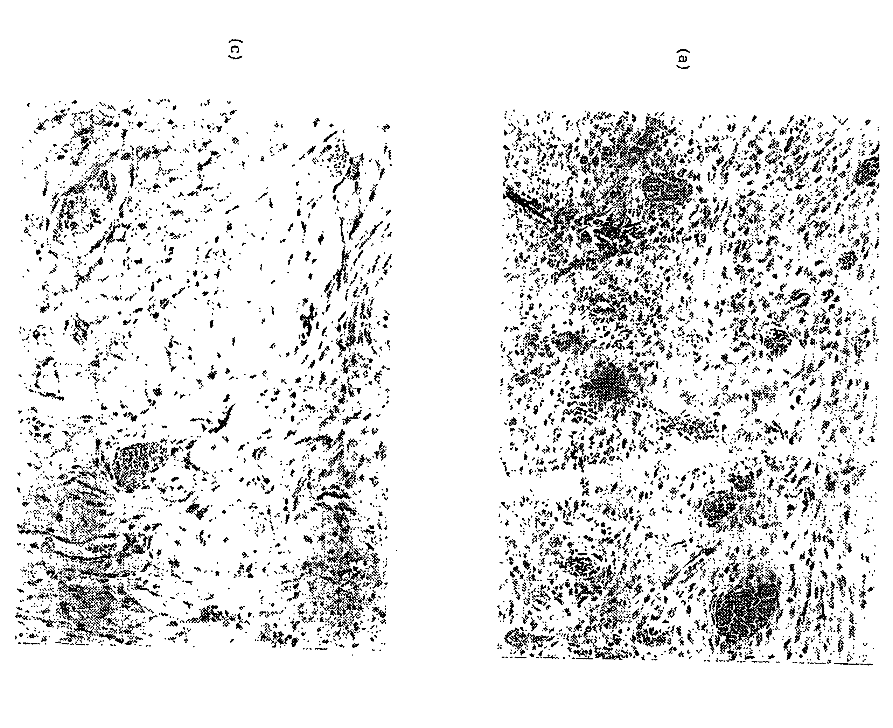

- **Fig. 1**: zeigt die Bildung von Gefäßen (Blutgefäßen) in Wunden, die mit einem Tissue-Faktor exprimierenden Vektor (a) transfiziert worden sind. (b) ist ein Vektor, der für einen "antisense" Tissue-Faktor kodiert, und (c) eine Kontrolle.

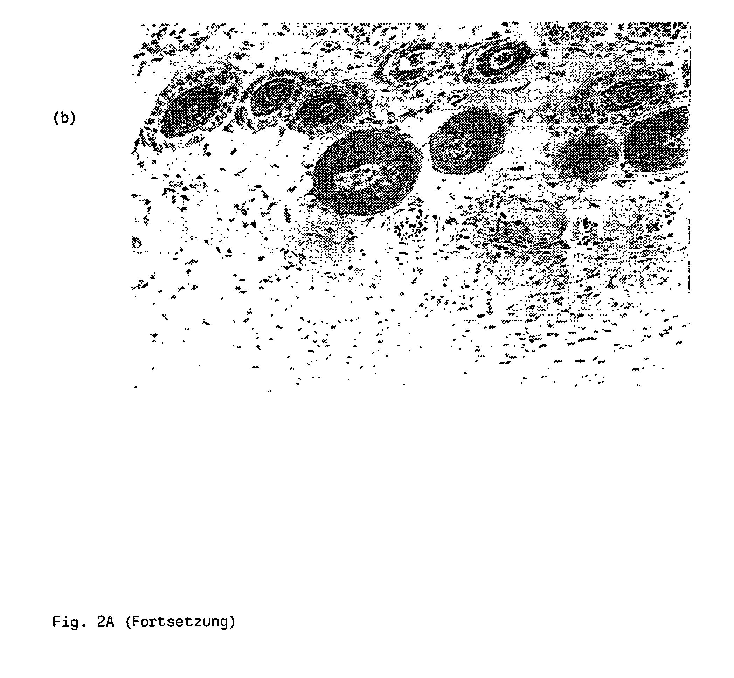

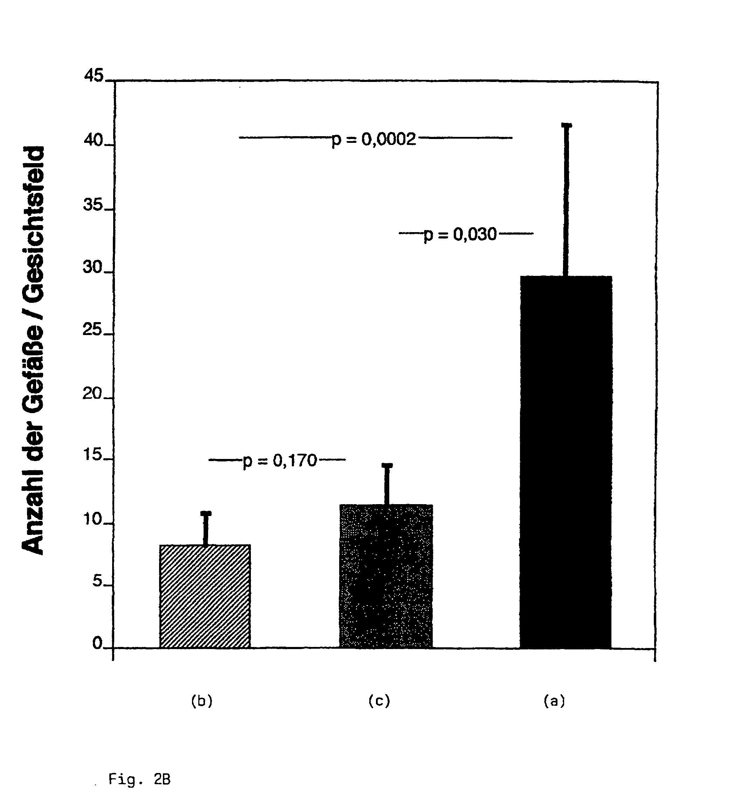

- **Fig. 2**: zeigt die Bildung von Gefäßen in Wunden, die mit einem Tissue-Faktor exprimierenden Vektor (a) transfiziert worden sind. (b) ist ein Vektor, der für einen "antisense" Tissue-Faktor kodiert, und (c) eine Kontrolle. Die Gefäße sind durch Hämatoxylin/Eosin-Färbung sichtbar gemacht (Fig. 2A). In Fig. 2B ist die Anzahl der Gefäße graphisch dargestellt.

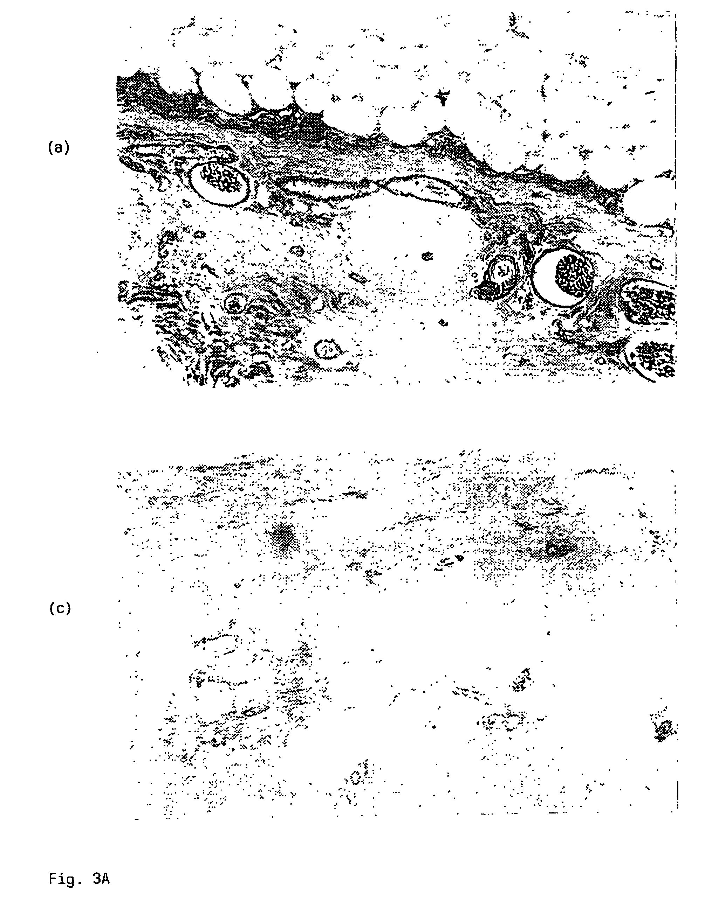

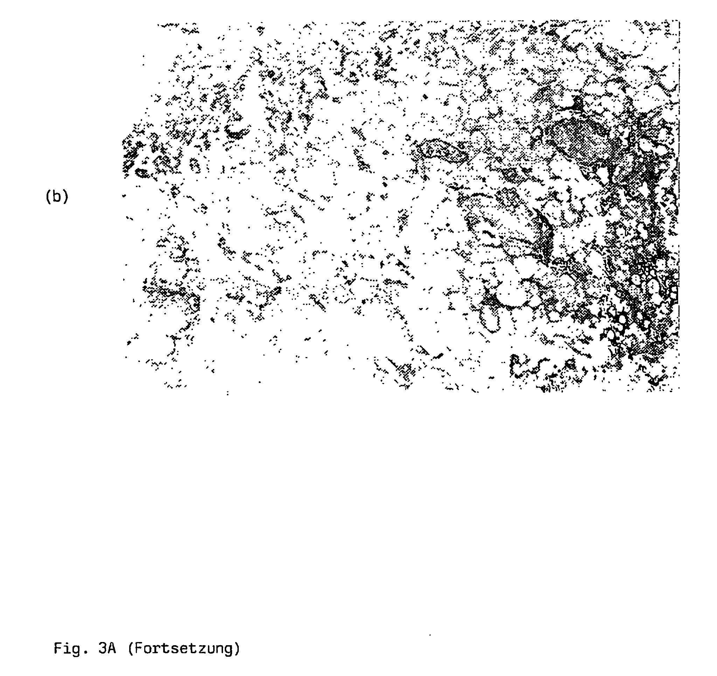

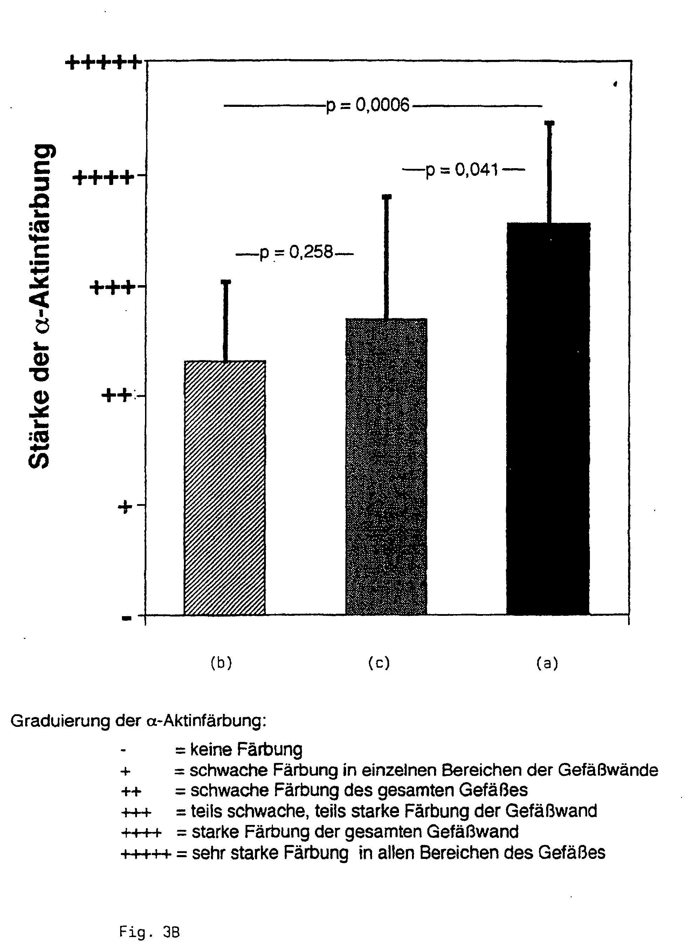

- **Fig. 3**: zeigt das Vorliegen glatter Muskelzellen in neu gebildeten Gefäßen in Wunden, die mit einem Tissue-Faktor exprimierenden Vektor (a) transfiziert worden sind. (b) ist ein Vektor, der für einen "antisense" Tissue-Faktor kodiert, und (c) eine Kontrolle. Die Muskelzellen sind durch eine α-Actin-Färbung sichtbar gemacht (Fig. 3A). In Fig. 3B ist die Stärke der Färbung graphisch dargestellt

Die vorliegende Erfindung wird durch das Beispiel erläutert.

### Beispiel: Herstellung eines Tissue-Faktor exprimierenden Plasmids und seine Verwendung zur Beeinflussung von Gefäßbildung, insbesondere zur Aktivierung von Gefäßbildung, ganz besonders bei Wundheilung

(A) in die BamHI-Stelle der "multiple-cloning site" von pcDNA3 (Invitrogen) wurde die gesamte translatierte Region (1,8 kb) des Maus-Tissue-Faktor-Gens integriert. Damit stand diese Region unter der Kontrolle des CMV-Promotors. Es wurde das Expressionsplasmid pcDNA3-TF erhalten. In gleicher Weise wurde in der EcoRI-Stelle der "multiple-cloning site" von pcDNA3 die kodierende Region (0,7 kb) des Maus-Tissue-Faktor-Gens in "antisense" Richtung integriert. Damit stand diese Region ebenfalls unter der Kontrolle des CMV-Promotors. Es wurde das Expressionsplasmid pcDNA3-TF-AS erhalten.
   Auf den Rücken von drei weiblichen NOD Mäusen (Bomholtgaard, Dänemark) wurden jeweils 6 mm "full thickness" Wunden im Abstand von 8 - 10 mm zueinander gesetzt. Diesen Wunden wurden Mischungen verabreicht, die 2µg pcDNA3-TF (a), pcDNA3-TF-AS (b) bzw. pcDNA3 (Kontrolle (c) und jeweils 12µg DOTAP-Transfektionsreagens (Boehringer Mannheim) enthielten. Die Wunden wurden mit Ohmann Opraflex bedeckt.
   Zum Nachweis der Bildung von Gefäßen (Blutgefäßen) in den Wunden wurden 6 bzw. 8 Tage nach Verabreichung der Mischungen jeweils 300µl Tinte (Nigrosin, Sigma) in die Schwanzvene der Mäuse injiziert. Danach wurden die Tiere getötet und die Hautpartien mit den Wunden unter dem Mikroskop untersucht.
   Es zeigte sich, daß bei Verabreichung eines Tissue-Faktor exprimierenden Vektors (a) Gefäße (Blutgefäße) in Wunden gebildet und somit Wundheilung gefördert wird. Ferner zeigte sich, daß ein "antisense" Tissue-Faktor die Bildung von Gefäßen inhibieren kann.
(B) Wie unter (A) beschrieben, wurden sechs NOD-Mäuse behandelt. Nach 6 bzw. 8 Tagen wurden die Tiere getötet und die entsprechenden Hautpartien unter dem Mikroskop untersucht, nachdem sie einer α-Actinfärbung (mit Sm-Actin Antikörper von Dianova) unterzogen worden waren.
   Es zeigte sich, daß in den gebildeten Gefäßen glatte Muskelzellen vorliegen.

## Patentansprüche

1. Verwendung von Tissue-Faktor oder einem Fragment davon zur Herstellung eines Arzneimittels zur gezielten therapeutischen Beeinflussung von Gefäßbildung durch Induktion einer lokalen Expression einer Tissue-Faktor Nukleinsäure oder durch lokale Applikation eines funktionellen Tissue-Faktor Proteins.

2. Verwendung nach Anspruch 1, wobei die Beeinflussung eine Aktivierung von Gefäßbildung ist.

3. Verwendung von Tissue-Faktor oder einem Fragment davon zur Herstellung eines Arzneimittels zur Beeinflussung der Wundheilung durch Induktion einer lokalen Expression einer Tissue-Faktor Nukleinsäure oder durch lokale Applikation eines funktionellen Tissue-Faktor Proteins.

4. Verwendung nach Anspruch 3, wobei es sich um die Wundheilung bei Diabetis mellitus, Vaskulitis, arterieller Verschlußkrankheit, chronischem venösem und infiziertem Ulcus, Innervationsstörungen, Dekubitus und Nahtschwächen bei Operationen handelt.

5. Verwendung nach Anspruch 1 oder 2, wobei es sich um die Gefäßbildung bei Arteriosklerose, M. Crohn und C. ulcerosa, diabetischer Retinopathie und tiefer Beinvenenthrombose/Ulcus cruris handelt.

6. Verwendung nach Anspruch 1 oder 2, wobei es sich um die Gefäßbildung zum Ersetzen von beeinträchtigten Gefäßen handelt.

7. Verwendung nach einem der Ansprüche 1-6, wobei der Tissue-Faktor oder ein Fragment davon als exprimierbare Nukleinsäure vorliegt.

8. Verwendung nach, Anspruch 7, wobei die Expression der Nukleinsäure transient ist.

9. Verwendung nach Anspruch 7 oder 8, wobei die Nukleinsäure eine DNA ist.

10. Verwendung nach einem der Ansprüche 7-9, wobei die Nukleinsäure unter der Kontrolle eines konstitutiven oder induzierbaren Promotors steht.

11. Verwendung nach einem der Ansprüche 7-10, wobei die Nukleinsäure in einem Sindbis-Virus-Replikon-Vektor vorliegt.

12. Verwendung nach einem der Ansprüche 7-10, wobei die Nukleinsäure unter der Kontrolle eines CMV- oder SV40-Promotors steht.

13. Verwendung nach einem der Ansprüche 1-12, wobei der Tissue-Faktor in einem Liposom oder auf einem Träger, insbesondere Goldpartikel, vorliegt.

14. Verwendung nach einem der Ansprüche 1-13, wobei der Tissue-Faktor in Kombination mit weiteren die Bildung von Gefäßen fördernden Faktoren vorliegt.

15. Verwendung nach Anspruch 14, wobei die Faktoren als exprimierbare Nukleinsäuren oder funktionelle Proteine vorliegen.

16. Verwendung nach Anspruch 14 oder 15, wobei einer der Faktoren VEGF ist.

17. Verwendung nach einem der Ansprüche 1-16, wobei der Tissue-Faktor in einer pharmazeutischen Zusammensetzung vorliegt.

## Claims

1. Use of tissue factor or a fragment thereof for the production of a medicament for the selective therapeutic influence of vascularization by inducing a local expression of a tissue factor nucleic acid or by a local application of a functional tissue factor protein.

2. Use according to claim 1, wherein the influence is an activation of vascularization.

3. Use of tissue factor or a fragment thereof for the production of a medicament for influencing wound healing by inducing a local expression of a tissue factor nucleic acid or by locally applying a functional tissue factor protein.

4. Use according to claim 3, wherein the wound healing is that in the case of diabetes mellitus, vasculitis, arterial occlusive disease, chronic venous and infected ulcer, innervation disturbances, decubitus and suture weakness in the case of operations.

5. Use according to claim 1 or 2, wherein the vascularization is that in the case of arteriosclerosis, Crohn's disease, C. ulcerosa, diabetic retinopathy and deep leg vein thrombosis/varicose ulcer.

6. Use according to claim 1 or 2, wherein the vascularization is that for replacing impaired vessels.

7. Use according to any of claims 1 to 6, wherein the tissue factor or a fragment thereof is present as expressible nucleic acid.

8. Use according to claim 7, wherein the expression of the nucleic acid is transient.

9. Use according to claim 7 or 8, wherein the nucleic acid is a DNA.

10. Use according to any of claims 7 to 9, wherein the nucleic acid is controlled by a constitutive or inducible promoter.

11. Use according to any of claims 7 to 10, wherein the nucleic acid is available in a sindbis virus replicon vector.

12. Use according to any of claims 7 to 10, wherein the nucleic acid is controlled by a CMV or SV40 promoter.

13. Use according to any of claims 1 to 12, wherein the tissue factor is available in a liposome or on a carrier, in particular gold particle.

14. Use according to any of claims 1 to 13, wherein the tissue factor is available in combination with other factors supporting the formation of vessels.

15. Use according to claim 14, wherein the factors are available as expressible nucleic acids or functional proteins.

16. Use according to claim 14 or 15, wherein one of the factors is VEGF.

17. Use according to any of claims 1 to 16, wherein the tissue factors is available in a pharmaceutical composition.

## Revendications

1. Utilisation d'un facteur de tissu ou d'un fragment de celui-ci pour la fabrication d'un médicament destiné à exercer une influence thérapeutique ciblée sur la vascularisation par induction d'une expression locale d'un acide nucléique de facteur de tissu ou par application locale d'une protéine fonctionnelle de facteur de tissu.

2. Utilisation selon la revendication 1, dans laquelle l'influence exercée est une activation de la vascularisation.

3. Utilisation du facteur de tissu ou d'un fragment de celui-ci pour la fabrication d'un médicament destiné à exercer une influence sur la cicatrisation par induction d'une expression locale d'un acide nucléique de facteur de tissu ou par application locale d'une protéine fonctionnelle de facteur de tissu.

4. Utilisation selon la revendication 3, dans laquelle il s'agit de la cicatrisation en cas de Diabetis mellitus, de vasculitis, d'occlusion artérielle, d'ulcère chronique veineux et infecté, de troubles de l'innervation, de décubitus et de faiblesses des sutures opératoires.

5. Utilisation selon la revendication 1 ou 2, dans laquelle il s'agit de la vascularisation en cas d'artériosclérose, de la maladie de Crohn et de C. ulcerosa, de rétinopathie diabétique et de thrombose veineuse de la jambe/d'Ulcus cruris.

6. Utilisation selon la revendication 1 ou 2, dans laquelle il s'agit de la vascularisation pour remplacer les vaisseaux endommagés.

7. Utilisation selon l'une des revendications 1 à 6, dans laquelle le facteur de tissu ou un fragment de celui-ci est présent sous forme d'acide nucléique exprimable.

8. Utilisation selon la revendication 7, dans laquelle l'expression de l'acide nucléique est transitoire.

9. Utilisation selon la revendication 7 ou 8, dans laquelle l'acide nucléique est un ADN.

10. Utilisation selon l'une des revendications 7 à 9, dans laquelle l'acide nucléique est sous contrôle d'un promoteur constitutif ou que l'on peut induire.

11. Utilisation selon l'une des revendications 7 à 10, dans laquelle l'acide nucléique est présent dans un vecteur de séquence de réplication du virus Sindbis.

12. Utilisation selon l'une des revendications 7 à 10, dans laquelle l'acide nucléique est sous contrôle d'un promoteur CMV ou SV40.

13. Utilisation selon l'une des revendications 1 à 12, dans laquelle le facteur de tissu est présent dans un liposome ou sur un support, en particulier une particule en or.

14. Utilisation selon l'une des revendications 1 à 13, dans laquelle le facteur de tissu est présent en combinaison avec d'autres facteurs favorisant la formation de vaisseaux.

15. Utilisation selon la revendication 14, dans laquelle les facteurs sont présents sous forme d'acides nucléiques exprimables ou de protéines fonctionnelles.

16. Utilisation selon la revendication 14 ou 15, dans laquelle l'un des facteurs est le VEGF (Facteur de Croissance Endothéliale Vasculaire).

17. Utilisation selon l'une des revendications 1 à 16, dans laquelle le facteur de tissu est présent dans une composition pharmaceutique.
